(19) Europäisches Patentamt
European Patent Office
Office européen des brevets

(11) Publication number: 0 050 671 B1

(12) EUROPEAN PATENT SPECIFICATION

(45) Date of publication of patent specification: 20.03.85

(51) Int. Cl.[4]: C 07 D 513/04, A 61 K 31/505

(21) Application number: 80900790.9

(22) Date of filing: 28.04.80

(86) International application number: PCT/JP80/00090

(87) International publication number: WO 81/03174 12.11.81 Gazette 81/27

# (54) THIAZOLO(3,2-a)PYRIMIDINE DERIVATIVES, PROCESS FOR PREPARING SAME, AND DRUG CONTAINING SAME.

(43) Date of publication of application:
05.05.82 Bulletin 82/18

(45) Publication of the grant of the patent:
20.03.85 Bulletin 85/12

(84) Designated Contracting States:
CH DE FR GB LI

(56) References cited:
JP-A-55 062 093

Journal of the American Chemical Society, vol. 64, November Nr. (1942), E.J. Masters et al., P2709 - 2711
Chemical Abstracts, vol.94, no. 5, February 2nd,1981, page 578, column 1, reference 30781p Columbus Ohio (US)

(73) Proprietor: TEIJIN LIMITED
11 Minami Honmachi 1-chome Higashi-ku
Osaka-shi Osaka-fu (JP)

(72) Inventor: OBA, Takeo
18-4, Tamadaira 3-chome
Hino-shi Tokyo 191 (JP)
Inventor: BANNAI, Kiyoshi
33-14, Asahigaoka 2-chome
Hino-shi Tokyo 191 (JP)
Inventor: TANAKA, Toshio
15-6, Tamadaira 5-chome
Hino-shi Tokyo 191 (JP)
Inventor: WATANABE, Kenzo
5-18, Tamadaira 3-chome
Hino-shi Tokyo 191 (JP)
Inventor: NARUCHI, Tatsuyuki
18-4, Tamadaira 3-chome
Hino-shi Tokyo 191 (JP)
Inventor: KOMORIYA, Keiji
5-18, Tamadaira 3 chome
Hino-shi Tokyo 191 (JP)
Inventor: KUROZUMI, Seizi
5-18, Tamadaira 5 chome
Hino-shi Tokyo 191 (JP)
Inventor: HOSHINA, Kenji
5-18, Tamadaira 3-chome
Hino-shi Tokyo 191 (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 050 671 B1

(74) Representative: **Votier, Sidney David et al**
**CARPMAELS & RANSFORD 43, Bloomsbury Square**
**London WC1A 2RA (GB)**

## Description

Technical field

The present invention relates to thiazolo [3,2-a] pyrimidine derivatives having excellent immunoregulating activities which are especially effective in curing such autoimmune diseases as nephritis and rheumatoid arthritis, and a process for the preparation thereof and an immunoregulating drug which contains said derivative as an active ingredient.

Background art

In recent years, immunoregulating therapy has come to be practiced to cure such autoimmune diseases as rheumatoid arthritis and generalized lupus erythematodes or malignant tumors and a variety of drugs have been developed. As an example of such drugs, the application of levamisole, which is a levorotatory isomer of tetramisole, to the drugs for immunotherapy of cancer and such autoimmune diseases as rheumatoid arthritis has due attention.

Though it has been reported that levamisole has efficacy in said diseases to a certain degree, it has no singularity in its efficacy and does not always display its efficacy satisfactorily either. Many immunoregulating drugs have been developed but most of them not only lack singularity in their remedial activities but also raise a problem of side effects, and no satisfactory drugs of this kind are available as yet.

Meanwhile, it is mentioned in the "Journal of American Chemical Society" vol. 64, pp. 2709—2712, 1942, that a derivative of barbituric acid is obtained from 2-aminothiazoline and diethyl malonate, and that this compound has a hypnotic action and an anesthetic action as well; however, nothing is mentioned in the journal as to other efficacy besides its hypnotic and anesthetic actions.

The inventors of the present invention have prepared novel thiazolo [3,2-a] pyrimidine derivatives which are novel barbituric acid derivative different from the derivatives of barbituric acid mentioned in the above journal and made a focussed study of the efficacy of this newly prepared compound. The result is a remarkable finding that it is a compound which has a specific immunoregulating action different from an action owned by levamisole, or the immunoregulating action based on a pharmacological action apart from hypnotic and anesthetic actions, and that, because of its low toxicity, the abovementioned thiazolo [3,2-a] pyrimidine derivatives are very useful for curing such autoimmune diseases as rheumatoid arthritis and nephritis.

Disclosure of invention

According to the present invention, there are provided thiazolo [3,2-a] pyrimidine derivatives having the formula (I)

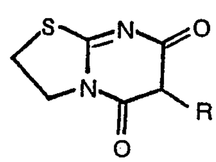

I

wherein R is:

a phenyl group substituted by a halogen atom, an alkyl group having 1 to 4 carbon atoms, or an alkyloxy group having 1 to 4 carbon atoms;

a benzyl group substituted in the phenyl moiety by a halogen atom, an alkyl group having 1 to 4 carbon atoms, or an alkyloxy group having 1 to 4 carbon atoms;

an alicyclic group having 3 to 7 carbon atoms or a phenethyl group.

The present invention also provides a process for the preparation of the derivatives according to claim 1, comprising cyclizing a compound of formula (II)

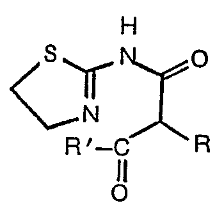

II

wherein R is as defined above and R′ is a halogen atom or a lower alkoxy by application of heat, and an immunoregulative drug containing a derivative of formula (I) as defined above as an immunoregulating active ingredient.

The group R may comprise such phenyl groups as p-chlorophenyl, o-chlorophenyl, m-chlorophenyl, p-fluorophenyl, m-fluorophenyl, o-fluorophenyl, p-bromophenyl, m-bromophenyl and o-bromophenyl which have a halogen atom as a substituent group; such phenyl groups as p-tolyl, o-tolyl, o-ethylphenyl, m-isopropylphenyl and p-butylphenyl which have a $C_1$—$C_4$ alkyl group as a substituent group; such phenyl groups as o-methoxyphenyl, p-ethoxyphenyl and m-propoxyphenyl, which have a

$C_1$—$C_4$ alkyloxy group as a substituent group; such benzyl groups as p-chlorobenzyl, o-chlorobenzyl, m-chlorobenzyl, p-fluorobenzyl, o-fluorobenzyl, m-fluorobenzyl, p-bromobenzyl, m-bromobenzyl and o-bromobenzyl which have a halogen atom as a substituent group on their phenyl rings; such benzyl groups as p-methylbenzyl, o-ethylbenzyl and m-isopropylbenzyl which have a $C_1$—$C_4$ alkyl group as a substituent group on their phenyl rings; and such benzyl groups as o-methoxybenzyl, p-ethoxybenzyl, m-propoxybenzyl, which have a $C_1$—$C_4$ alkyloxy group as a substituent group on their phenyl rings.

As an alicyclic group, there are cyclopropyl, cyclobutyl, cyclopentyl, cycloheptyl, cyclohexyl, and 2-methylcyclopropyl.

Of these groups mentioned above, it is preferable to select R from such phenyl groups or benzyl groups as p-chlorophenyl, o-chlorophenyl, m-chlorophenyl, p-chlorobenzyl, o-chlorobenzyl, m-chlorobenzyl, p-fluorophenyl, m-fluorophenyl, o-fluorophenyl, p-fluorobenzyl, o-fluorobenzyl, m-fluorobenzyl, p-bromophenyl, m-bromophenyl, o-bromophenyl, p-bromobenzyl, m-bromobenzyl, and o-bromobenzyl which have a chlorine, fluorine, or bromine atom as a substituent on their phenyl rings respectively, a cyclohexyl group or a phenethyl group.

p-Chlorobenzyl, o-chlorobenzyl, p-chlorophenyl and o-chlorophenyl are especially preferable.

The thiazolo [3,2-a] pyrimidines of the present invention may be of the enol form which have the same pharmacological activity.

Also said derivatives may take the form of acid addition salt of an inorganic acid or an organic acid. Inorganic acid, are e.g. hydrochloric acid, hydrobromic acid, and hydroiodic acid; organic acids, are e.g. acetic acid, propionic acid, lactic acid, and maleic acid.

The derivatives of the thiazolo [3,2-a] pyrimidine of formula (I) can be prepared by cyclizing a compound of formula (II) by application of heat

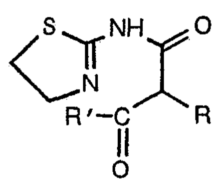

II

R of the above formula (II) is the same R as in the aforementioned formula (I).

R′ is a halogen atom such as bromine, iodine or chlorine, or a lower alkyloxy group such as methoxy or ethoxy.

The cyclization of a compound of formula (II) by application of heat may be conducted either with the use of a solvent or in the absence of a solvent. In the cyclization in which a solvent is used, toluene, xylene, cumene, cymene, tetralin, decalin, diethylene glycol dimethyl ether, diethylene glycol diethyl ether, diethylene glycol dibutyl ether, diphenyl ether, dimethyl sulfoxide, dimethylformamide and nitrobenzene, may be mentioned as recommendable solvents. The amount of such solvents to be used is usually in the range of 1 to 1,000 times the molar quantity of the starting material.

Though the reaction temperature and time vary depending upon the starting material, presence or absence of a solvent, kind of the solvent, the desired thiazolo [3,2-a] pyrimidine derivative is usually obtained by conducting the reaction at 80 to 320°C for 1 minute to 48 hours.

A compound of formula (II) can be obtained, for instance, by reacting 2-aminothiazoline of formula (III)

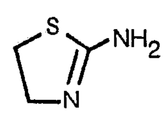

III

with a compound formula (IV) while heating

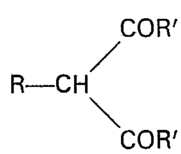

IV

In the abovementioned formula (IV), R is the same R as defined in the aforementioned formula (I) and R′ is the same R′ as defined in the aforementioned formula (II).

The abovementioned reaction may be carried out with the use of a solvent or no solvent. In case where a solvent is used, the kinds of solvents and their amounts to be used can be the same solvents and amounts as mentioned for cyclizing a compound of formula (II) by heating.

As for the method for the preparation of a thiazolo [3,2-a] pyrimidine derivative of the present invention, it is preferable first to heat a compound of formula (III) with a compound of formula (IV) to obtain a compound of formula (II), and without isolating the resulting intermediate of formula (II), further letting the reaction continue by heating to obtain the thiazolo [3,2-a] pyrimidine derivative of the present invention.

The thiazolo [3,2-a] pyrimidine derivatives of formula (I) are especially used for curing each autoimmune diseases as rheumatoid-arthritis and nephritis and is administered orally, or nonorally by way such as rectally, subcutaneously, or intramuscularly.

For oral administration, the drug is prepared in the form of a solid preparation or a liquid preparation. As a solid preparation, there are a tablet, pill, powder, and granule. In preparing these solid preparations, one or more thiazolo [3,2-a] pyrimidine derivatives are used as active ingredients and they are mixed with at least one inactive diluent such as commonly used calcium carbonate, potato starch, alginic acid and lactose. The preparation is carried out according to the ordinary method and additional agents other than a diluent, for instance, such a lubricant as magnesium stearate may be contained in them.

Liquid preparation for oral administration use contain pharmaceutically permissible emulsifier, solvent, suspension, syrup and commonly used inactive diluents such as water and liquid paraffin.

These preparations contain other auxiliary agents such as a wetting agent, auxiliary suspending agent, sweetening agent, flavoring agent, aromatic, and preservative in addition to said inactive diluents.

Also these liquid preparations may be enclosed in capsules made from such an assimilable substance as gelatin.

As a solid preparation for rectum administration use, a suppository, which contains one or more active principles and is prepared according to a publicly known method, may be mentioned.

Preparations to be administered nonorally include aseptically prepared aqueous and nonaqueous solution, suspension, and emulsion. As solvent or suspending agent for nonaqueous preparation use, there are propylene glycol, polyethylene glycol, vegetable oil such as olive oil, and injectable organic ester such as ethyl oleate. These preparations may also contain such auxiliary agents as a preservative, wetting agent, emulsifying agent, and dispersing agent. These preparations can be made aseptic by filtration through a bacterial filter, or by addition of a bactericide, or by irradiation. An aseptic solid preparation prepared beforehand may be used by dissolving it in aseptic water or aseptic injectable solvent immediately before its administration.

The dosage of the thiazolo [3,2-a] pyrimidine derivative is 0.1 to 50 mg/kg, especially preferable in the range of 0.5 to 20 mg/kg; however, the dosage varies depending upon the condition of the disease and age of a patient and the method of administration.

The present invention is illustrated for further details by the following examples.

Example 1:

A mixture consisting of 1.0 g of 2-aminothiazoline and 2.4 g of cyclohexyl diethyl malonate was made to react in a stream of nitrogen for 30 minutes while heating at 180°C. After the reaction product was allowed to cool down to room temperature, it was chromatographed on a column of silica gel and was developed with ethyl acetate-benzene (1:4) to obtain 2.01 g of desired 6-cyclohexyl-5H-2,3,6,7-tetrahydro-5,7-dioxothiazolo [3,2-a] pyrimidine (yield: 80%). The properties of this matter were as follows:

Melting point: 250 to 251°C.

IR ($\nu_{max}^{KBr}$) $cm^{-1}$:

3200—2500 (enol form), 1655, 1525, 1426, 1321, 705

NHR ($\delta_{TMS}^{DMSO-d6}$):

1.0—2.0 (11 H, m), 3.50 (2H, t, J=8Hz), 4.33 (2H, t, J=8Hz), 11.0 (1H, br, $D_2O$, disappeared).

Example 2

A mixture consisting of 1.02 g of 2-aminothiazoline and 3.0 g of p-chlorophenyl diethyl malonate was heated at 180°C for 30 minutes to carry out the reaction. After the reaction product was allowed to cool down to room temperature, 30 ml of ether was added thereto, and the precipitated crystals were filtered off to obtain 1.7 g of desired 6-p-chlorophenyl-5H-2,3,6,7-tetrahydro-5,7-dioxothiazolo [3,2-a] pyrimidine (yield: 63%). The properties of this matter were as follows:

Melting point: 275°C

IR ($\nu_{max}^{KBr}$) $cm^{-1}$:

3100—2600 (enol form), 1645, 1590, 1528, 1437, 1390

NMR ($\delta_{TMS}^{DMSO-d6}$):

3.75 (2H, t, J=8Hz), 4.43 (2H, t, J=8Hz), 7.4 (4H, m), 11.5 (1H, br).

Example 3

A mixture consisting of 1.5 g of 2-aminothiazoline and 4.5 g of p-chlorobenzyl diethyl malonate was made to react in a stream of nitrogen for 2 hours while heating at 180°C. 8 ml of diphenyl ether was added to the obtained solid reaction product and the mixture was further made to react in a stream of nitrogen while heating at 220°C for 2 hours. The product resulting from the reaction was allowed to cool down to room temperature, chromatographed on a column of silica gel and eluted with chloroform-methanol (96:4) to obtain 1.8 g of desired 6-p-chlorobenzyl-5H-2,3,6,7-tetrahydro-5,7-dioxothiazolo [3,2-a] pyrimidine (yield: 38%). The properties of this matter were as follows:

Melting point: 273.5—275.5°C.

IR ($\nu_{KBr}^{max}$) $cm^{-1}$:

3100—2300 (enol form), 1655, 1640, 1625, 1545, 1445, 1403, 1289, 1110, 1098, 800.

NMR ($\delta_{TMS}^{DMSO-d6}$):

3.48 (br, t, J=8Hz), 3.52 (s), 4.28 (2H, br, t, J=8Hz), 7.26 (4H, s), 9.30 (1H, br).

Example 4

A mixture consisting of 0.5 g of 2-aminothiazoline and 1.5 g of o-chlorobenzyl diethyl malonate was made to react in a stream of nitrogen for 1.5 hours while heating at 170°C. The reaction product thus obtained was chromatographed on a column of silica gel and eluted with chloroform-methanol (97—90: 3—10) to give 0.86 g of desired 6-(o-chlorobenzyl)-5H-2,3,6,7-tetrahyro-5,7-dioxothiazolo [3,2-a] pyrimidine (yield: 45%). The properties of this matter were as follows:

Melting point: 265—274°C.

IR ($\nu_{KBr}^{max}$) $cm^{-1}$:

3100—2300 (enol form), 1650, 1620, 1528, 1440, 1400, 1100, 1038, 748

NMR ($\delta_{TMS}^{DMSO-d6}$):

3.52 (t, J=8Hz), 3.62 (2H, s), 4.33 (2H, t), 7.2 (4H, m), 11.63 (1H, br).

Example 5

A mixture consisting of 2.0 g of 2-aminothiazoline and 5.8 g of phenethyl diethyl malonate was made to react in a stream of nitrogen for 1.5 hours while heating at 180°C. 8 ml of diphenyl ether was added to thus obtained oily product and the mixture was again made to react in a stream of nitrogen for 1.5 hours while heating at 220°C. The obtained reaction product was put to column chromatography on silica gel and eluted with chloroform-methanol (90—95:10—5) to give 2.74 g of desired 5H-2,3,6,7-tetrahydro-5,7-dioxo-6-phenethylthiazolo [3,2-a] pyrimidine (yield: 51%). The properties of this matter were as follows:

Melting point: 253—263°C,

IR ($\nu_{KBr}^{max}$) $cm^{-1}$:

3200—2300 (enol form), 1640, 1605, 1515, 1400, 1150, 1100, 752.

NMR ($\delta_{TMS}^{DMSO-d6}$):

2.56 (4H, s), 3.47 (2H, t, J=8Hz), 4.27 (2H, t, J=8Hz), 7.20 (5H, s).

Example 6

A mixture consisting of 0.5 g of 2-amino-thiazoline and 1.3 g of p-methylbenzyl diethyl malonate was made to react in a stream of nitrogen for 2 hours while heating at 180°C. The reaction product was then put to column chromatography on silica gel and eluted with chloroform methanol (99:1) to obtain 0.6 g of desired 5H-2,3,6,7-tetrahydro-6-p-methylbenzyl-5,7-dioxothiazolo [3,2-a] pyrimidine (yield: 44%). The properties of this matter were as follows:

Melting point: 267—268.5°C.

IR ($\nu_{max}^{KBr}$) $cm^{-1}$:

3200—2610 (enol form), 1640, 1615, 1530, 1440, 1400, 1287, 1096, 802, 786

NMR ($\delta_{TMS}^{DMSO-d6}$):

2.28 (3H, s), 3.53 (2H, s), 3.53 (2H, t, J=8Hz), 4.38 (2H, t, J=8Hz), 7.28 (4H, s), 10.7 (1H, br).

Example 7

5H-2,3,6,7-tetrahydro-6-p-methoxybenzyl-5,7-dioxothiazolo [3,2-a] pyrimidine was obtained according to the same method as Example 6. The properties of this matter were as follows:

Yield: 39%

Melting point: 228—230°C.

IR ($\nu_{max}^{KBr}$) $cm^{-1}$:

3400—2650 (enol form), 1640, 1610, 1530, 1505, 1435, 1280, 1240, 1100, 1022, 800

NMR ($\delta_{TMS}^{DMSO-d6}$):

3.57 (2H, s), 3.59 (2H, t, J=8Hz), 3.76 (3H, s), 4.38 (2H, t, J=8Hz), 6.87 (2H, d, J=9Hz), 7.27 (2H, d, J=9Hz), 11.3—11.7 (1H, br, $D_2O$, disappeared).

Example 8

5H-2,3,6,7-tetrahydro-6-o-methoxybenzyl-5,7-dioxothiazolo [3,2-a] pyrimidine was obtained according to the same method as Example 6. The properties of this matter were as follows:

Yield: 55%

Melting point: 259—260.5°C.

IR ($\nu_{max}^{KBr}$) cm$^{-1}$:

3400—2570 (enol form), 1653, 1595, 1515, 1446, 1345, 1293, 1208, 1090, 777, 758, 742, 722

NMR ($\delta_{TMS}^{DMSO-d6}$):

3.56 (2H, t, J=8Hz), 3.61 (2H, s), 3.88 (3H, s), 4.40 (2H, t, J=8Hz), 6.7—7.5 (4H, m), 11.0 (1H, br).

Example 9

6-p-fluorobenzyl-5H-2,3,6,7-tetrahydro-5,7-dioxothiazolo [3,2-a] pyrimidine was obtained according to the same method as Example 6. The properties of this matter were as follows:

Yield: 58%

Melting point: 267—268°C.

IR ($\nu_{max}^{KBr}$) cm$^{-1}$:

3500—2630 (enol form), 1625, 1540, 1500, 1442, 1405, 1287, 1212, 1105, 820, 790, 737

NMR ($\delta_{TMS}^{DMSO-d6}$):

3.54 (2H, t, J=8Hz), 3.59 (2H, s), 4.37 (2H, t, J=8Hz), 6.9—7.6 (4H, m), 11.5—11.9 (1H, br, $D_2O$, disappeared).

Example 10

5H-2,3,6,7-tetrahydro-6-p-methoxyphenyl-5,7-dioxothiazolo [3,2-a] pyrimidine was obtained according to the same method as Example 6. The properties of this matter were as follows:

Yield: 18%

Melting point: 266—268°C.

IR ($\nu_{max}^{KBr}$) cm$^{-1}$:

3400—2650 (enol form) 1642, 1600, 1570, 1555, 1425, 1395, 1290, 1245, 1180, 1138, 1062, 1028, 952, 860, 835, 821, 776, 742

NMR ($\delta_{TMS}^{DMSO-d6}$):

3.55 (2H, t, J=7Hz), 3.75 (3H, s), 4.35 (2H, t, J=7Hz), 6.7—7.6 (4H, m), 11.3—11.7 (1H, br, $D_2O$, disappeared).

Example 11

5H-2,3,6,7-tetrahydro-6-o-methoxyphenyl-5,7-dioxothiazolo [3,2-a] pyrimidine was obtained according to the same method as Example 6. The properties of this matter were as follows:

Yield: 27%

Melting point: 256—258°C.

IR ($\nu_{max}^{KBr}$) cm$^{-1}$:

3400—3100 (enol form), 1620, 1592, 1510, 1400, 1275, 1140, 1110, 1060, 1045, 1023, 790, 747

NMR ($\delta_{TMS}^{DMSO-d6}$):

3.54 (2H, t, J=7Hz), 3.68 (3H, s), 4.35 (2H, t, J=7Hz), 6.9—7.5 (4H, m), 10.6 (1H, br).

Example 12

5H-2,3,6,7-tetrahydro-5,7-dioxo-6-p-bromophenylthiazolo [3,2-a] pyrimidine was obtained according to the same method as Example 6, having the following properties:

Yield: 48%

Melting point: 284—287°C

IR ($\nu_{max}^{KBr}$) cm$^{-1}$:

3200—2200 (enol form), 1640, 1540, 1430, 1380, 1300, 1225, 1138, 1058, 1008, 950, 825

NMR ($\delta_{TMS}^{DMSO-d6}$):

3.55 (2H, t, J=8Hz), 4.35 (2H, t, J=8Hz), 7.50 (4H, s), 10.5 (1H, br).

Example 13

In this example the immunoregulating actions of the undermentioned compounds obtained in Examples 1 to 5 and Example 9 were examined with the results shown herein:

6-cyclohexyl-5H-2,3,6,7-tetrahydro-5,7-dioxothiazolo [3,2-a] pyrimidine (hereinafter referred to as medicine A), 6-p-chlorophenyl-5H-2,3,6,7-tetrahydro-5,7-dioxothiazolo [3,2-a] pyrimidine (hereinafter referred to as medicine B), 6-p-chlorobenzyl-5H-2,3,6,7-tetrahydro-5,7-dioxothiazolo [3,2-a] pyrimidine (hereinafter referred to as medicine C), 6-(o-chlorobenzyl)-5H-2,3,6,7-tetrahydro-5,7-dioxothiazolo [3,2-a] pyrimidine (hereinafter referred to as medicine D), 5H-2,3,6,7-tetrahydro-5,7-dioxo-6-phenethylthiazolo [3,2-a] pyrimidine (hereinafter referred to as medicine E), and 6-p-fluorobenzyl-5H-2,3,6,7-tetrahydro-5,7-dioxothiazole [3,2-a] pyrimidine (hereinafter referred to as medicine F).

(i) Effect on delayed-type hypersensitivity:

The effect of the medicines was estimated with an index of delayed-type hypersensitivity which is caused by injecting red sheep blood cells as antigen into the pads of mice (see the method described by P. H. Lagrange, G. B. Mackaness, and T. L. Mille, Journal of Experimental Medicine, Vol. 139, pp. 1529—1539, 1974; Immunology, Vol. 34, p. 363, 1978).

Groups consisting of 4 or 5 C3H/He male mice (7 weeks old) were used in the experiments. The mice were sensitized by injecting $10^6$ red sheep blood cells suspended in 0.2 ml of physiological saline intraveneously into the tail vein. Then the mice were administered orally with the abovementioned medicines in doses mentioned in Table 1 immediately after the sensitization, 1 day after the sensitization, 2 days after the sensitization, and 3 days after the sensitization respectively. 24 hours after the oral administration of the medicines made 3 days after the sensitization, $10^8$ sheep red blood cells suspended in 25 $\mu l$ of physiological saline were injected subcutaneously dermically into the right hind foot pad of the mice to induce a hypersensitivity. 24 hours after the induction of the allergic reaction, the thickness of the pad was measured with a micrometer to determine the increase in thickness (foot pad reaction 0.1 mm) as compared with the thickness of the pad before the subcutaneous injection of $10^8$ sheep red blood cells thus to know the effect of the medicines on the delayed-type hypersensitivity.

For the purpose of comparison, experiments were conducted with the mice which were not given the medicines and with those which were administered with levamisole and D-penicillamine respectively in the place of the medicines of the present invention. The results of these experiments are shown in Table 1.

TABLE 1

| | Medicine | Dose (mg/kg) | Number of cases | Foot pad reaction (0.1 mm) |
|---|---|---|---|---|
| Experiment 1 | Control | — | 8 | 3.1±0.5 |
| | Medicine C | 0.3 | 4 | 4.1±0.2 |
| | " | 1 | " | 4.9±0.5* |
| | " | 3 | 5 | 4.3±0.4 |
| | " | 10 | " | 4.1±0.7 |
| | Levamisole | 1 | " | 4.3±0.4 |
| | " | 3 | " | 4.6±0.3 |
| | " | 10 | 4 | 5.1±0.3* |
| | D-penicillamine | 1 | 5 | 5.0±0.6 |
| | " | 3 | " | 5.3±0.4** |
| | " | 10 | " | 3.7±0.3 |
| Experiment 2 | Control | | 8 | 4.7±0.7 |
| | Medicine C | 1 | 5 | 7.2±0.5* |
| | " | 5 | " | 6.7±0.7 |
| | " | 25 | " | 6.5±0.4 |
| | Medicine B | 1 | 4 | 5.3±0.8 |
| | " | 5 | " | 5.7±0.5 |
| | " | 25 | " | 5.7±1.0 |
| | Medicine D | 1 | 5 | 6.9±0.9 |
| | " | 5 | 4 | 6.9±1.0 |
| | " | 25 | 5 | 6.1±1.2 |
| | Levamisole | 1 | 4 | 7.6±0.4* |
| | " | 5 | " | 5.9±0.3 |
| | " | 25 | 5 | 6.8±1.0 |
| Experiment 3 | Control | | 4 | 4.7±1.1 |
| | Medicine A | 1 | " | 6.3±1.0 |
| | " | 10 | " | 6.2±1.3 |
| | Medicine E | 1 | " | 4.0±0.8 |
| | " | 10 | " | 5.8±0.7 |

TABLE 1 (contd.)

| | Medicine | Dose (mg/kg) | Number of cases | Foot pad reaction (0.1 mm) |
|---|---|---|---|---|
| Experiment 4 | Control | | 5 | 3.0±0.1 |
| | Medicine F | 1 | " | 5.7±0.5** |
| | " | 3 | " | 3.9±0.4 |

*: $P<0.05$,
** $P<0.01$

It is evident from Table 1 that the medicines according to the present invention are as effective as levamisole and D-penicillamine in increasing the thickness of the hind foot pad of mice (foot pad reaction), in other words, in accelerating the delayed-type hypersensitivity, which proves that the medicines of the present invention have a function to enhance the immunizing action.

(ii) Effect on lymphocyte blast cell transformation

(a) The experiments were made according to the method described in the Journal of Immunology, Vol. 122, pp 1—7, 1979 to study the effect of the medicines on the highly exasperated state of immunizing function created by adding a mitogen, which is a cell proliferating factor, to spleen cells obtained from mice to accelerate the cell division.

In the experiments, spleen cells obtained from normal BAL B/c female mice (7 to 8 weeks old) were suspended in a culture medium (RPMI-1640) containing 5% fetus calf serum (FCS). Then a mitogen (concanavalin A or lipopolysaccharide) and the medicine dissolved in dimethyl sulfoxide were added to the culture medium of spleen cells ($10^5$/100 $\mu$l). The cells were incubated in the microculture plate in a 95% air, 5% $CO_2$ atmosphere at 37°C. After the 3-day incubation was over, the 3H-thymidine (0.5 $\mu$l Ci/20 $\mu$l) was added thereto and the incubation was conducted for another 18 hours. 3H-thymidine incorporated into the cells was determined with a liquid scintillation counter.

For the purpose of comparison, experiments were conducted likewise with the cells to which the medicine or mitogen was not added and with those to which levamisole and D-penicillamine were added respectively in the place of the medicines of the present invention. The results of these experiments are shown in Table 2.

TABLE 2

| Medicine | Concentration (μg/ml) | Mitogen (μg/ml) | | | | |
|---|---|---|---|---|---|---|
| | | | Concanvalin A | | Lipopolysaccharide | |
| | | 0 | 0.25 | 5 | 10 | 50 |
| Medicine C | 0 | 193±17 | 4985±802 | 22460±2742 | 1996±432 | 2644±413 |
| | 1.0 | 143±17 | 3653±192 | 17919±657 | 2167±633 | 1654±243 |
| | 2.5 | 164±4 | 4753±1004 | 13686±1260 | 1371±198 | 1052±82 |
| | 5.0 | 218±40 | 4484±876 | 8568±2009 | 1529±400 | 737±312 |
| Levamisole | 0 | 687±59 | 8835±648 | 24733±1043 | 4906±320 | 4132±508 |
| | 10 | 1377±157 | 11780±922 | 93325±12561 | 15359±2944 | 16667±862 |
| | 25 | 3142±388 | 20365±1337 | 139837±26679 | 48044±6048 | 62257±1144 |
| | 50 | 3473±126 | 24163±1332 | 79916±15075 | 63961±7405 | 69523±4231 |
| D-pencillamine | 0 | 687±59 | 8835±648 | 24733±1043 | 4906±320 | 4132±508 |
| | 1 | 490±81 | 5171±768 | 18657±2176 | 1672±261 | 2670±473 |
| | 10 | 399±26 | 4115±559 | 21824±565 | 1342±266 | 1349±176 |
| | 25 | 359±52 | 3932±513 | 31558±3340 | 1446±320 | 1515±390 |

It is apparent from Table 2 that levamisole has a tendency to accelerate the incorporation of 3H-thymidine in both the case where mitogen is not added and the case where mitogen added, while medicine C of the present invention does not exert much influence on the incorporation of 3H-thymidine in the case where mitogen is not added but is has a tendency to strongly suppress the incorporation of 3H-thymidine in the case where mitogen is added. D-penicillamine shows a tendency somewhat similar to that of medicine C but is rather weak.

The above fact concludes that levamisole has the immunostimulative effect on both cells which have a normal immune activity and cells which have an abnormally stimulated immune activity: on the contrary, the medicines of the present invention exercises no influence on the cells which have a normal immune activity but has an immunosuppressive effect only on cells which have an abnormally stimulated immune activity. Therefore, it is confirmed that the medicines of the present invention are drugs that have a very singular immunoregulative activity.

(b) The experiments were carried out according to the same method as the preceding (a) with the use of NZB/WF, female mice which were made to be spontaneously attacked with systemic lupus erythematosus. The results are shown in Table 3.

TABLE 3

| Medicine | Concentration (μg/ml) | Mitogen (μg/ml) | | | | |
|---|---|---|---|---|---|---|
| | | 0 | Concanavalin A | | Lipopolysaccharide | |
| | | | 0.25 | 5 | 10 | 50 |
| Medicine C | 0 | 2137±164 | 16327±1282 | 33411±5150 | 20965±1300 | 26212±2662 |
| | 1.0 | 1721±364 | 12449±1215 | 27807±4082 | 19319±1386 | 22170±1736 |
| | 2.5 | 1478±240 | 10498±966 | 22191±865 | 14828±1829 | 14197±2169 |
| | 5.0 | 1433±133 | 9291±1064 | 19532±115 | 15893±2168 | 10867±2430 |
| Levamisole | 0 | 2880±736 | 15925±1788 | 32465±2205 | 25136±321 | 28834±1544 |
| | 10 | 5411±257 | 42026±4849 | 65800±7832 | 56054±3101 | 61717±3538 |
| | 25 | 11775±373 | 61492±5661 | 94057±418 | 89072±3024 | 127316±12956 |
| | 50 | 18498±3276 | 53202±6127 | 119202±15483 | 109322±9136 | 151299±3331 |
| D-pencillamine | 0 | 2880±736 | 15925±1788 | 32465±2205 | 25136±321 | 28834±1544 |
| | 1 | 2923±105 | 16999±519 | 35597±5483 | 22175±2705 | 27587±3280 |
| | 10 | 1853±211 | 25081±2500 | 32047±4296 | 16788±1497 | 23217±728 |
| | 25 | 1880±648 | 29798±178 | 37527±4740 | 15557±293 | 15880±1491 |

As shown in Table 3, levamisole accelerates the incorporation of 3H-thymidine as in the case of the normal mice in the experiments conducted in (a) disregard to whether mitogen is added or not added to the spleen cells obtained from the mice made diseased, while medicine C of the present invention vigorously suppress the 3H-thymidine incorporation when mitogen is added to the spleen cells obtained from the mice made diseased and also suppress the 3H-thymidine incorporation when mitogen is not added. D-penicillamine also shows a tendency somewhat similar to that of medicine C but is rather weak.

It is also concluded from the above fact that, different from levamisole, medicine C of the present invention has a specific effect to suppress the abnormally accelerated immunogenic action of the cells. Thus it is confirmed that the medicines of the present invention has a very singular immunoregulative activity.

From the preceding (i) and (ii), it is evident that the medicines of the present invention have such singular immunoregulative activities as to accelerate the immunity, to exercise no influence to the cells which have a normal immunizing function, and to suppress the abnormally accelerated immunity.

Example 14

This examples shows that the medicines of the present invention have the low toxicity.

The acute toxicity of the medicine was determined by use of groups of 6 male ICR mice, 6 weeks old, according to an ordinary method.

The results are shown in Table 4.

TABLE 4

| Medicine | Administration | L D50 (g/kg) |
|---|---|---|
| 6-p-chlorobenzyl-5H-2,3,6,7-tetrahydro-5,7-dioxothiazolo [3,2-a] pyrimidine | Oral administration | 4 or more |

It is apparent from Table 4 that the toxicity of the medicines of the present invention is very low.

Example 15

Tablets consisting of the following components per tablet were prepared:

| | |
|---|---|
| Active ingredient (6-p-chlorobenzyl-5H-2,3,6,7-tetrahydro-5,7-dioxothiazolo [3,2-a] pyrimidine) | 200 mg |
| Lactose | 280 mg |
| Potato starch | 80 mg |
| Polyvinyl pyrrolidone | 11 mg |
| Magnesium stearate | 5 mg |

A mixture of the active ingredient, lactose and potato starch was homogeneously moistened with 20% ethanol solution of polyvinyl pyrrolidone, sieved through a 2.0 mm mesh sieve, dried at 45°C, and sieved again through a 1.5 mm mesh sieve. The granules thus obtained were mixed with magnesium stearate and compressed to give tablets.

The compound obtained in Example 3 was used as an active ingredient to represent the medicines of the present invention.

Example 16

Hara gelatin capsules consisting of the following components per capsule were prepared:

| | |
|---|---|
| Active ingredient (6-p-chlorobenzyl-5H-2,3,6,7-tetrahydro-5,7-dioxothiazolo [3,2-a] pyrimidine) | 200 mg |
| Micro-crystalline cellulose | 195 mg |
| Amorphous silicic acid | 5 mg |

The finely powdered active ingredient, microcrystalline cellulose and uncompressed amorphous silicic acid were mixed thoroughly and contained in hard gelatin capsules.

Example 17

5 ml ampules consisting of the following components per capsule were prepared:

| | | |
|---|---|---|
| Active ingredient (6-p-chlorobenzyl-5H-2,3,6,7-tetrahydro-5,7-dioxothiazolo [3,2-a] pyrimidine) | | 200 mg |
| Polyethylene glycol 600 | | 200 mg |
| Distilled water | To make a total of | 5.0 ml |

Polyethylene glycol and the active ingredient were dissolved in water in an atmosphere of nitrogen, boiled, cooled in an atmosphere of nitrogen, and distilled. Pre-treated water was added to thus obtained solution to make a prescribed volume and filtered under the aseptic conditions. The preparation was conducted in the diffused light.

The filling up was conducted in a stream of nitrogen and the sterilization was carried out at 121°C for 20 minutes.

Industrial application

Since the derivatives of thiazolo [3,2-a] pyrimidine of the present invention have singular and excellent immunoregulating activities, they can be used for curing such autoimmune diseases as rheumatoid arthritis, systemic lupus erythematodes, nephritis, nephrosis, Behoet disease, Chrohn disease, idiopathic ulcerative colitis, polyneuritis, fibrosis of the lung, or autoimmune hemolytic anemia, uveitis.

**Claims**

1. Thiazolo [3,2-a] pyrimidine derivatives having the formula (I)

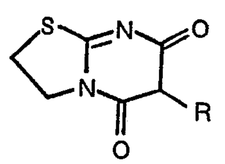

I

wherein R is:

a phenyl group substituted by a halogen atom, an alkyl group having 1 to 4 carbon atoms, or an alkyloxy group having 1 to 4 carbon atoms;

a benzyl group substituted in the phenylmoiety by a halogen atom, an alkyl group having 1 to 4 carbon atoms, or an alkyloxy group having 1 to 4 carbon atoms;

an alicyclic group having 3 to 7 carbon atoms or a phenethyl group.

2. Derivatives according to claim 1, wherein R is:

a phenyl group substituted by a chlorine, fluorine or bromine atom;

a benzyl group substituted in the phenyl moiety by a chlorine, fluorine, or bromine atom;

a cyclohexyl group; or

a phenethyl group.

3. Derivatives according to claim 2, wherein R is:

a chlorophenyl group; or

a benzyl group substituted by a chlorine atom in the phenyl ring.

4. A process for the preparation of the derivatives according to claim 1, comprising cyclizing a compound of formula (II)

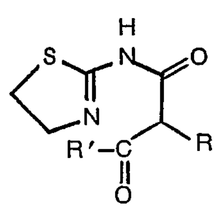

II

wherein R is as defined in claim 1 and R′ is a halogen atom or a lower alkoxy group, by application of heat.

5. A process according to claim 4, wherein R′ is a methoxy or ethoxy group.

6. An immunoregulative drug containing a derivative according to any one of claims 1 to 3 as an immunoregulating active ingredient.

**Patentansprüche**

1. Thiazolo-[3,2-a]-pyrimidinderivate der Formel (I)

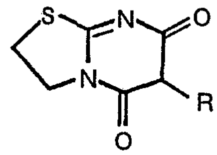

I

worin R eine Phenylgruppe, die durch ein Halogenatom, eine Alkylgruppe mit 1 bis 4 Kohlenstoffatomen oder eine Alkoxygruppe mit 1 bis 4 Kohlenstoffatomen substituiert ist, eine Benzylgruppe, die im Phenylring durch ein Halogenatom, eine Alkylgruppe mit 1 bis 4 Kohlenstoffatomen oder eine Alkoxygruppe mit 1 bis 4 Kohlenstoffatomen substituiert ist, eine alicyclische Gruppe mit 3 bis 7 Kohlenstoffatomen oder eine Phenethylgruppe bedeutet.

2. Derivate nach Anspruch 1, worin R eine durch ein Clor-, Fluor, oder Bromatom substituierte Phenylgruppe, eine durch ein Chlor-, Fluor- oder Bromatom im Phenylring substituierte Benzylgruppe, eine Cyclohexylgruppe oder eine Phenethylgruppe bedeutet.

3. Derivate nach Anspruch 2, worin R eine Chlorphenylgruppe oder eine im Phenylring durch ein Chloratom substituierte Benzylgruppe bedeutet.

4. Verfahren zur Herstellung der Derivate nach Anspruch 1, dadurch gekennzeichnet, daß man eine Verbindung der Formel (II),

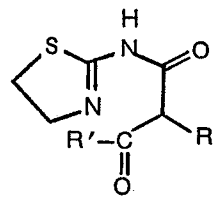

II

worin R die in Anspruch 1 angegebene Bedeutung besitzt und R' ein Halogen oder eine niedere Alkoxygruppe bedeutet, durch Anwendung von wärme zyklisiert.

5. Verfahren nach Anspruch 4, dadurch gekennzeichnet, daß R' eine Methoxy- oder Ethoxygruppe ist.

6. Immunoregulatives Arzneimittel, enthaltend ein Derivat nach einem der Ansprüche 1 bis 3 als immunoregulierenden Wirkstoff.

**Revendications**

1. Dérivés de la thiazolo [3,2-a]-pyrimidine, ayant la formule (I):

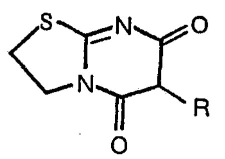

I

dans laquelle R représente: un groupe phényle substitué par un atome d'halogène, un groupe alkyle ayant 1 à 4 atomes de carbone ou un groupe alkyloxy ayant 1 à 4 atomes de carbone;

un groupe benzyle substitué sur le noyau phényle per un atome d'halogène, un groupe alkyle ayant 1 à 4 atomes de carbone ou un groupe alkyloxy ayant 1 à 4 atomes de carbone;

un groupe alicyclique ayant 3 à 7 atomes de carbone; ou

un groupe phénéthyle.

2. Dérivés selon la revendication 1, dans lesquels R représente;

un groupe phényle substitué par un atome de chlore, de fluor ou de brome;

un groupe benzyle substitué, sur le noyau phényle, par un atome de chlore, de fluor ou de brome;

un groupe cyclohexyle; ou

un groupe phénéthyle.

3. Dérives selon la revendication 2, dans lesquels R représente:

un groupe chlorophényle; ou

un groupe benzyle substitué par un atome de chlore sur le noyau phényle.

4. Procédé de préparation des dérivés selon la revendication 1, comprenant la cyclisation par chauffage d'un composé de formule (II):

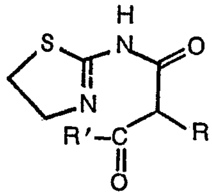 II

dans laquelle R est comme défini à la revendication 1 et R′ est un atome d'halogène ou un groupe alcoxy inférieur.

5. Procédé selon la revendication 4, dans lequel R′ est un groupe méthoxy ou éthoxy.

6. Médicament immunorégulateur, contenant un dérivé selon l'une quelconque des revendications 1 à 3 comme principe actif, immunorégulateur.